Europäisches Patentamt

(19) European Patent Office

Office européen des brevets

(11) Numéro de publication: **0 102 279**

**A1**

(12) # DEMANDE DE BREVET EUROPEEN

(21) Numéro de dépôt: **83401581.0**

(22) Date de dépôt: **29.07.83**

(51) Int. Cl.³: **C 12 M 1/00**

(30) Priorité: **12.08.82 FR 8214063**

(43) Date de publication de la demande:
**07.03.84 Bulletin 84/10**

(84) Etats contractants désignés:
**AT BE CH DE GB IT LI NL**

(71) Demandeur: **CHAMBRE D'AGRICULTURE DE L'AISNE**
**Place Edouard Herriot**
**F-02006 Laon Cédex(FR)**

(72) Inventeur: **Berry, Jean-Luc**
**3 rue Georges Gouigoux**
**F-60200 Compiegne(FR)**

(74) Mandataire: **Durand, Yves Armand Louis et al,**
**Cabinet Z. Weinstein 20, Avenue de Friedland**
**F-75008 Paris(FR)**

(54) Procédé et installation de production de biogaz à partir d'un effluent liquide organique, tel que par exemple le lisier et échangeur thermique comme moyen nécessaire pour la mise en oeuvre dudit procédé ou de ladite installation.

(57) L'invention concerne un procédé et une installation de production de biogaz à partir d'un effluent liquide organique.

L'installation comprend un fermenteur (1) étanche, combiné de préférence avec un échangeur thermique (80), le fermenteur (1) contenant des bactéries fixées sur un support organisé (2) formé par une pluralité d'éléments poreux (22) immergés à canaux de passage traversant sensiblement parallèles disposés en piles successives (23, 24) espacées. Des moyens (8) de récupération de biogaz sont prévus et comprennent une ou plusieurs conduites (8a, 8b) débouchant au sommet de chaque compartiment (10, 12, 14, 16, 18, 20) pour collecter le biogaz libéré se dégageant entre les piles (23, 24) d'éléments poreux (22).

L'invention permet d'éviter tout colmatage dans le fermenteur ou échangeur thermique avec un excellent rendement en biogaz et une accessibilité indépendante des compartiments du fermenteur sans arrêter le fonctionnement de l'installation.

./...

FIG. 1

-1-

## Procédé et installation de production de biogaz à partir d'un effluent liquide organique, tel que par exemple le lisier et échangeur thermique comme moyen nécessaire pour la mise en œuvre dudit procédé ou de ladite installation

La présente invention concerne essentiellement un procédé et une installation de production de biogaz à partir d'un effluent liquide organique, tel que par exemple le lisier et un échangeur thermique comme moyen nécessaire pour la mise en œuvre du procédé ou de l'installation.

On connaît déjà diverses installations de production de biogaz du type ci-dessus travaillant en discontinu ou même en continu.

Dans les installations fonctionnant en discontinu, on entasse tout d'abord des déchets organiques, tels que par exemple du fumier, dans une cuve où il subit une fermentation aérobie fortement exothermique puis on ajoute du purin ou analogue au contenu de cette cuve qui subit alors une fermentation anaérobie donnant naissance à du méthane sous forme de biogaz (gaz ayant une forte proportion de $CO_2$) que l'on recueille pour des utilisations diverses. Ces installations ont l'avantage de permettre de travailler avec des concentrations élevées en matières organiques mais elles exigent des manipulations pénibles et donnent une production cyclique de gaz.

D'un autre côté, les installations en fermentation continue doivent travailler avec des matières organiques pompables donc plus diluées mais, dans de telles installations, les manipulations désagréables sont réduites au minimum et la production de gaz y est régulière.

Cependant, toutes les installations connues jusqu'à présent, qu'elles soient du type à fonctionnement discontinu ou continu, présentent de mauvais rendements. En effet, la fermentation anaérobie état lente et dégageant peu de chaleur, sa température a tendance à s'abaisser de sorte que le débit de gaz est très réduit. Et si l'on veut maintenir artificiellement cette température à un niveau convenable, il est nécessaire de récourir à une dépense d'énergie qui correspond le plus souvent au tiers voire à la moitié du méthane produit à partir du biogaz, et ceci malgré une bonne isolation thermique.

La fermentation anaérobie peut être réalisée dans des fermenteurs ou digesteurs à bactéries libres avec ou sans décantation, agités ou non de façon à conserver au mieux une partie de la flore bactérienne et à la mettre efficacement au contact du substrat.

Un tel type d' installation à fermenteur à bactéries libres agité est décrit dans la demande de brevet français RAMBAUD N° 2 480 874.

Cette installation comprend un fermenteur de construction spéciale mais qui n'est utilisable que dans le cas où on précède la fermentation anaérobie par une

-3-

fermentation aérobie, ce qui constitue une limitation très importante à l'emploi d'une telle installation car la fermentation aérobie détruit une partie non négligeable des micro-organismes réalisant la fermentation anaérobie de sorte que l'on aboutit à une réduction importante de la production de biogaz.

D'autre part, cette installation RAMBAUD ne permet pas d'éviter un colmatage rapide dès que le substrat comporte quelques éléments organisés non solubles comme des boues, de la paille, des poils, etc. On observera à ce sujet que dans l'installation RAMBAUD il est prévu un dispositif à serpentin qui favorise un tel colmatage.

On observera en outre qui si l'on désire éviter la fermentation aérobie qui est préjudiciable à la bonne réalisation de la fermentation anaérobie qui seule réalise la production de biogaz, l'installation RAMBAUD n'est plus apte à permettre le maintien de la température convenable lors de la fermentation anaérobie. A ce sujet, on doit souligner que le serpentin ne peut aboutir au chauffage convenable de l'effluent liquide car il se colmate très rapidement par les éléments organisés non solubles.

D'une manière plus générale aucun échangeur de chaleur connu, qui soit à plaque, tubulaire ou à spirale ne convient pour l'application à des effluents liquides organiques qui contiennent nécessairement des éléments organiques et non solubles comme des boues, de la paille, des poils, etc... entraînant un colmatage très rapide, un encrassement par incrustation de dépôt et/ou décantation de boue. Enfin, tous les échangeurs connus sont inaccessibles en cours de fonctionnement pour enlever les dépôts.

D'autre part, il est à souligner que l'on a tenté d'utiliser des fermenteurs à bactéries fixées sur support qu'il soit mobile (lit fluidisé par exemple) ou fixe en vrac (garnissant type colonne à distiller par exemple) pour améliorer le rendement en biogaz.

Cependant, tous ces dispositifs à bactéries fixées sont parcourus par un flux liquide vertical cheminant le plus souvent de bas en haut, c'est-à-dire dans le même sens que le biogaz produit.

Il en résulte un certain nombre de contraintes et d'inconvénients tels que :
    - forte consommation d'énergie pour la circulation ;
    - méthodicité impossible à respecter par la suite des recyclages inévitables ;
    - colmatage rapide dès que le substrat comporte quelques éléments organisés non solubles (boues, pailles, poils etc...) ;
    - érosion de la biomasse fixée par les supports mobiles ;
    - inaccessibilité en cours de marche et sans vidange complète.

Finalement, les installations à fermenteurs à bactéries fixées sur support n'ont donc pu être également utilisées en pratique.

La présente invention a donc pour but de remédier aux inconvénients de la technique antérieure en fournissant une solution qui permette d'utiliser la technique des bactéries fixées sur support en vue d'accroître grandement le rendement en biogaz tout en évitant un colmatage par les éléments organisés non solubles du substrat qui est un effluent liquide organique.

Avantageusement, cette solution doit être de conception
particulièrement simple et permettre un continu réel
y compris dans l'alimentation, une récupération
du biogaz dans un chemin différent du flux liquide
de sorte à favoriser la séparation du biogaz produit
par un cheminement horizontal et méthodique du liquide.
D'autre part, cette solution doit aussi avantageusement
permettre une récupération de la chaleur de l'effluent
en évitant une consommation de biogaz comme cela
est en général préconisé et en permettant une accessibilité sans vidange du liquide ni interruption du
processus. Aussi, cette solution doit permettre le
remplacement possible du support pendant et sans perturber le fonctionnement et un contrôle appropriés.

Cette solution consiste selon la présente invention
en un procédé de production de biogaz à partir d'un
effluent liquide organique, tel que par exemple le
lisier, du type comprenant une fermentation anaérobie
à une température convenable dans un fermenteur étanche contenant des bactéries fixées sur support
organisé par alimentation de préférence continue du
fermenteur en effluent liquide sans le remplir complètement, soutirage du fermenteur, de préférence continu,
de l'effluent fermenté et la récupération du biogaz produit de l'effluent, caractérisé en ce qu'on réalise
le fermenteur étanche avec au moins un compartiment
de fermentation, chaque compartiment étant pourvu
d'une pluralité d'élément poreux immergés à canaux
de passage traversant sensiblement parallèles,
disposés en piles successives espacées entre elles
d'un espace prédéterminé et ayant les canaux en
position horizontale et dans le sens d'écoulement
naturel de l'effluent liquide dans chaque compartiment;
on fait circuler l'effluent liquide horizontalement
dans ledit fermenteur, on procède à une agitation,

de préférence permanente, de l'effluent liquide avantageusement en réalisant des pulsations et on récupère par le sommet de fermenteur le biogaz libéré par la fermentation anaérobie et se dégageant entre les piles d'éléments poreux.

Avantageusement, selon l'invention, on préchauffe l'effluent liquide par échange thermique dans un échangeur thermique disposé à l'extérieur du fermenteur tandis que l'ensemble fermenteur et échangeur est de préférence disposé à l'intérieur d'une enceinte extérieure thermiquement isolante.

La présente invention concerne aussi une installation de production de biogaz à partir d'un effluent liquide organique, tel que par exemple le lisier, du type comprenant un fermenteur étanche, contenant des bactéries fixées sur un support organisé, pourvu de moyens d'alimentation et de moyens de soutirage en effluent liquide, lequel ne remplit pas complètement le fermenteur, et de moyens de récupération du biogaz produit de l'effluent, caractérisée en ce que le fermenteur étanche comprend au moins un compartiment de fermentation pourvu d'une pluralité d'éléments poreux immergés à canaux de passage traversant sensiblement parallèles, disposés en piles successives espacées entre elles d'un espace prédéterminé et ayant leurs canaux en position horizontale et dans le sens d'écoulement naturel de l'effluent liquide circulant horizontalement dans le fermenteur; de préférence de moyens d'agitation, avantageusement permanente, de l'effluent liquide avantageusement en réalisant des pulsations, lesdits moyens de récupération de biogaz comportant un ou plusieurs conduits débouchant au sommet de chaque compartiment pour collecter le biogaz libéré se dégageant entre les piles d'éléments poreux.

Selon un mode de réalisation préféré, l'installation selon l'invention comprend un échangeur thermique disposé à l'extérieur du fermenteur dont le circuit de fluide réfrigérant est parcouru par l'effluent liquide à introduire dans le fermenteur et le circuit de fluide chauffant est parcouru par l'effluent liquide sortant du fermenteur tandis qu'avantageusement l'ensemble fermenteur-échangeur thermique est disposé à l'intérieur d'une enceinte fermée thermiquement isolante.

Il est à noter que la conception de l'installation complète est entièrement nouvelle et qu'ainsi le fermenteur et/ou l'échangeur thermique sont de conception entièrement nouvelle.

Ainsi, l'invention concerne aussi l'échangeur thermique en soi et plus particulièrement comme moyen nécessaire à la mise en oeuvre du procédé précité ou de l'installation.

La structure de cette installation selon l'invention de conception entièrement nouvelle sera maintenant décrite en référence aux dessins annexés dans lesquels:

La figure 1 est une vue générale d'une installation selon la présente invention schématique de dessus avec le dessus de l'enceinte isolante enlevé ainsi que le toit du fermenteur et le couvercle de l'échangeur thermique pour apercevoir leur structure interne;

La figure 2 est une vue de dessus agrandie du fermenteur représenté à la figure 1;

La figure 3 est une vue en coupe selon la ligne de trace III-III de la figure 2;

La figure 4 est une vue de détail des moyens d'agitation du fermenteur selon la ligne de trace IV-IV de la figure 2;

La figure 5 est une vue en coupe selon la ligne de trace V-V de la figure 1;

La figure 6 est une vue en élévation avec la partie frontale de l'enceinte isolante enlevée;

La figure 7 est une vue agrandie de dessus de l'échangeur avec couvercle et partie de l'enceinte isolante enlevés pour voir la structure interne de l'échangeur thermique;

La figure 8 est une vue en coupe selon la ligne de trace VIII-VIII de la figure 7;

La figure 9 est une vue de détail de dessus du dispositif d'agitation de l'échangeur thermique hors de l'échangeur;

La figure 10 est une vue de détail de dessus de la position du dispositif d'agitation à l'intérieur de l'échangeur thermique en fin de course d'agitation de celui-ci au niveau des cloisons immergées de l'échangeur thermique;

La figure 11 est une vue selon la flèche XI de la figure 8;

La figure 12 représente en coupe partielle une variante de réalisation de l'échangeur thermique avec un joint hydraulique; et

La figure 13 est une vue frontale analogue à la figure 11 du mode de réalisation avec joint hydraulique représenté à la figure 12.

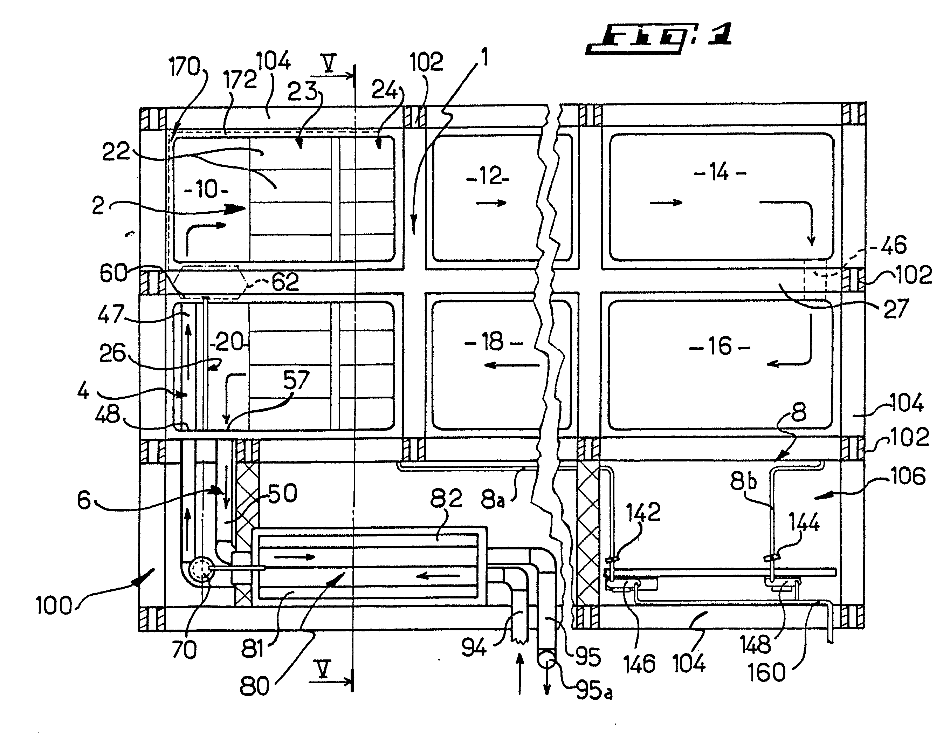

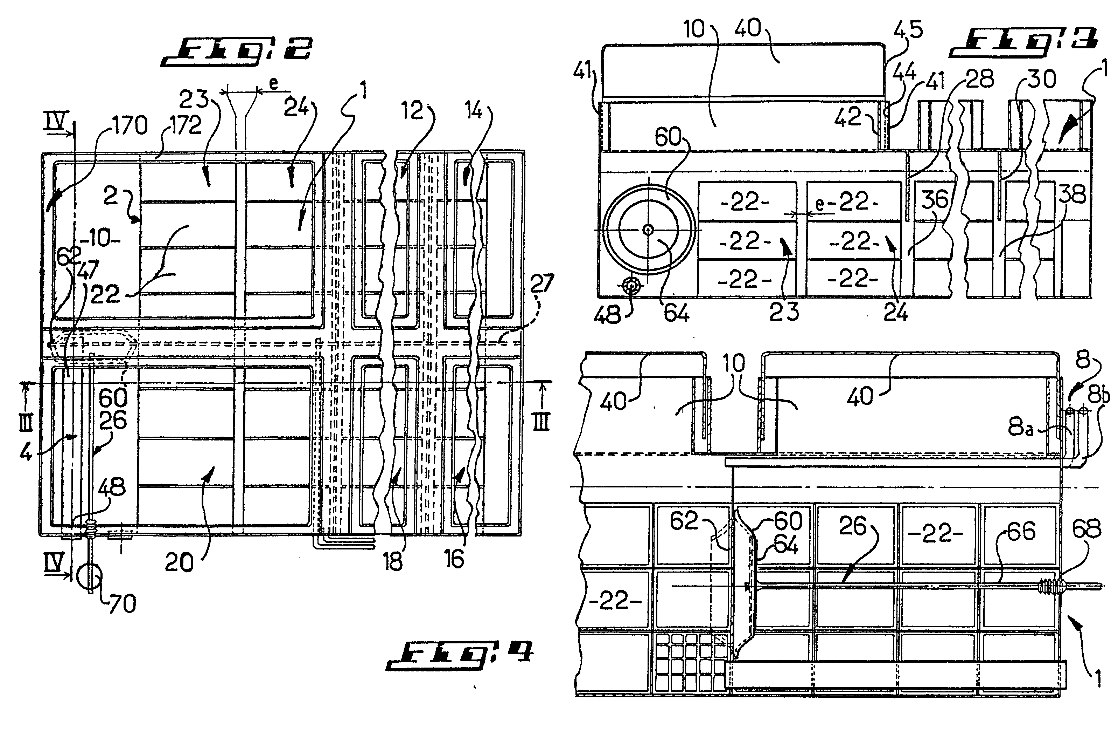

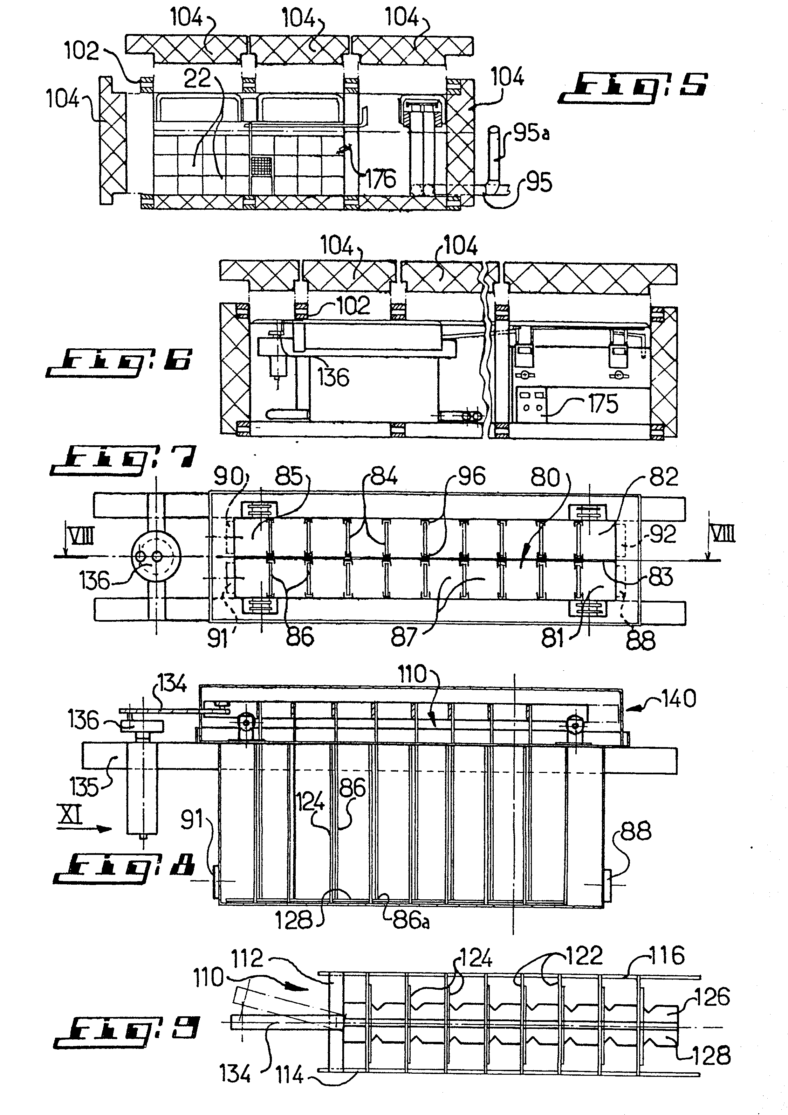

En référénce aux figures 1 à 5, une installation selon la présente invention pour une production de biogaz à partir d'un effluent organique liquide, tel que par exemple le lisier, est du type comprenant un fermenteur 1 étanche, contenant des bactéries fixées sur un support organisé 2, pourvu de moyens d'alimentation 4 et de moyens de soutirage 6 en effluent liquide lequel ne remplit pas complètement le fermenteur 1 comme cela est clairement visible à la figure 3, et des moyens 8 de récupération de biogaz produit de l'effluent.

Cette installation est caractérisée en ce que le fermenteur étanche comprend au moins un compartiment 10, 12, 14, 16, 18, 20 de fermentation pourvu d'une pluralité d'éléments poreux 22 immergés à canaux de passage traversants sensiblement paralléles disposés en piles successives 23, 24 espacées entre elles d'un espace e prédéterminé et ayant leurs canaux en position horizontale et dans le sens d'écoulement naturel de l'effluent liquide circulant horizontalement dans le fermenteur comme cela se conçoit bien à partir de la considération des figures 1 et 2 et comme matérialisé par les flèches de circulation de l'effluent liquide.

Cette installation comprend aussi des moyens 26 d'agitation, de préférence permanente, de l'effluent liquide avantageusement en réalisant des pulsations tandis que les moyens 8 de récupération de biogaz comportent un ou plusieurs conduits tels que 8a, 8b débouchant au sommet de chaque compartiment pour collecter le biogaz libéré se dégageant entre les piles d'éléments poreux comme cela apparait clairement à partir de la considération des figures 1, 2 et 4 principalement.

Selon un mode de réalisation préféré, tel que représenté, le fermenteur 1 comprend une pluralité de compartiments 10, 12, 14, 16, 18, 20 successifs indépendants dont la paroi commune (par exemple 28, 30) entre deux compartiments successifs est au moins en partie ouverte vers le bas définissant ainsi une ouverture telle que 36, 38, respectivement, de passage de l'effluent liquide au fond du compartiment, chaque compartiment, tel que le compartiment 10, comportant un toit 40 amovible propre de fermeture étanche reposant sur les parois, telles que 41, 42 de son compartiment 10 associé.

Selon une caractéristique particulièrement avantageuse, chaque compartiment 10, 12, 14, 16, 18, 20 comme représenté et clairement visible à la figure 3, comprend un joint hydraulique, tel que 44 formé par la rigole définie entre les parois 41, 42 espacées surmontant le compartiment 10, destiné à recevoir du liquide et dans laquelle rigole 44 viennent se placer les bords latéraux rabattus 45 en forme de jupe du toit amovible 40 de manière à réaliser une fermeture parfaitement étanche au fluide et surtout au gaz. Tous les compartiments sont réalisés de la même manière comme cela se conçoit bien à partir de la figure 3, ce qui permet une ouverture et donc une accessibilité indépendante des compartiments en cours de fonctionnement.

Selon le mode de réalisation représenté actuellement préféré du fermenteur, celui-ci est formé par une cuve sensiblement parallélépipédique comprenant une cloison médiane 27 de séparation étanche du fermenteur en deux zones et une ou plusieurs cloisons semi-immergées formant les parois communes précitées telles que 28, 30 représentées à la figure 3 ouvertes vers le bas définissant la pluralité de compartiments successifs précités dont chaque compartiment présente son toit propre tel que 40.

Selon ce mode de réalisation actuellement préféré, le compartiment 10 d'entrée de l'effluent et le compartiment 20 de sortie de l'effluent sont situés de part et d'autre de la cloison médiane 27 qui comporte naturellement une ouverture 46 de communication entre les compartiments 14 et 16 les plus éloignés des compartiments d'entrée 10 et de sortie 20 pour une circulation correcte de l'effluent liquide entre les divers compartiments. Cette disposition permet de limiter la longueur du fermenteur mais il est naturellement concevable d'avoir tous les compartiments disposés successivement l'un à la suite de l'autre en chaîne. Selon cette disposition, ainsi les moyens d'alimentation 4 comprennent une conduite 47 passant au travers de deux ouvertures 48, 49 prévues respectivement dans la paroi de la cuve définissant le fermenteur 1 et la cloison médiane 27 pour déboucher librement dans le compartiment 10. De même, les moyens de soutirage 6 comprennent une conduite 50 disposée à proximité de la conduite 47 et communiquant avec le compartiment de sortie 20 par une ouverture 51 prévue à cet effet dans la paroi externe de la cuve 1.

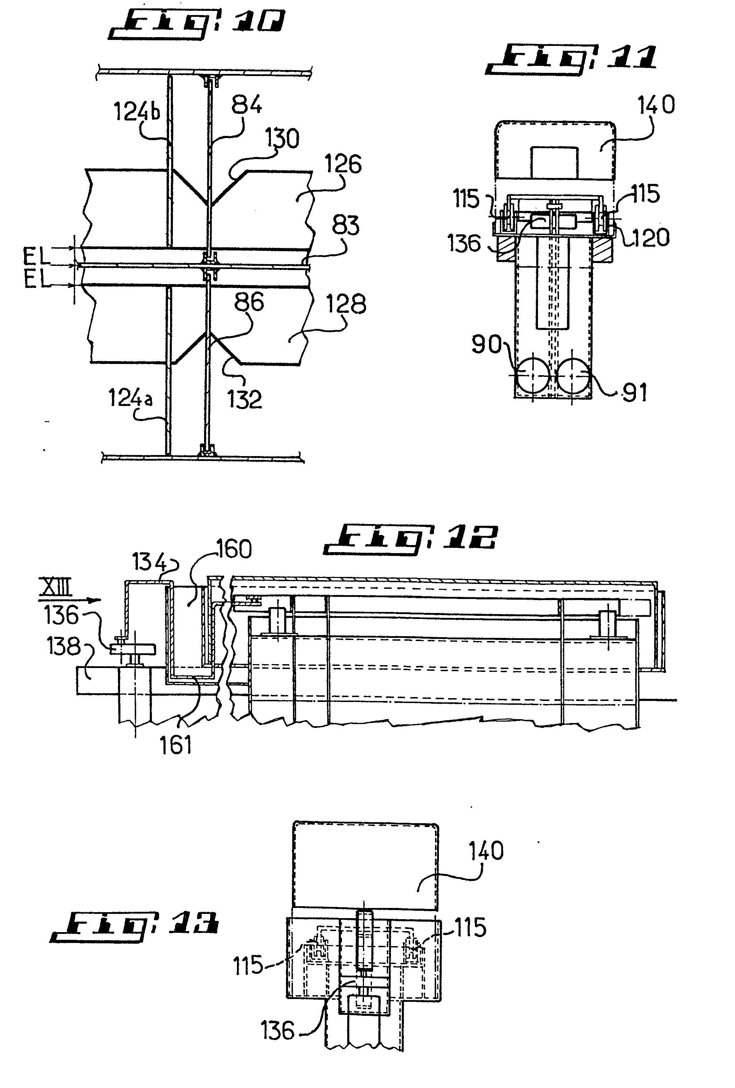

Les moyens d'agitation par pulsation précités dans le fermenteur sont de préférence formés par une membrane 60 souple déformable qui ferme une ouverture 62 prévue dans la cloison médiane 27 entre le compartiment d'entrée 10 et le compartiment de sortie 20. Cette membrane 60 est rendue rigide en son centre par un disque 64 fixé de façon étanche sur la membrane 60 qui est lui-même mû suivant son axe par un mécanisme approprié par exemple comprenant une bielle 66 traversant la paroi de la cuve du fermenteur 1 de façon étanche par exemple au moyen d'un soufflet 68 et qui peut être entraîné par un motoréducteur 70 ou analogue extérieur à la cuve 1. On observera que à la figure 4 on a représenté en traits pleins la position en fin de course dans le compartiment de sortie 20 du fermenteur 1 et en traits mixtes les positions intermédiaires ainsi que la position extrême en fin de course dans le compartiment d'entrée 10. On conçoit ainsi que cette structure forme des moyens d'agitation 26 induisant des pulsations amont-aval dans tout le fermenteur 1.

Selon une caractéristique préférée de la présente invention, l'installation selon l'invention comprend en outre un échangeur thermique 80 disposé à l'extérieur du fermenteur 1 dont le circuit 81 de fluide réfrigérant est parcouru par l'effluent liquide à introduire dans le fermenteur par les moyens d'alimentation 4 et le circuit 82 de fluide chauffant est parcouru par l'effluent liquide sortant du fermenteur 1 par les moyens de soutirage 6. De préférence, l'ensemble fermenteur 1-échangeur thermique 80 est disposé à l'intérieur d'une enceinte 100 fermée thermiquement isolante qui est par exemple constituée d'une structure porteuse 102 formant une charpente qui peut être en bois lamellé-collé ayant une très faible conductivité thermique et supportant l'isolant proprement dit 104 très épais, rigide et porteur englobant totalement l'installation, y compris la face au contact du sol. Cet isolant 104 peut être réalisé à partir de laine de verre ou de mousse de polyuréthane ou tout matériau analogue remplissant la même fonction. L'isolant 104 est avantageusement composé de différentes plaques que l'on voit particulièrement bien à la figure 6 fixées sur la structure porteuse 102 et amovibles pour la plupart de façon à permettre l'accès indépendant aux différents compartiments et organes. Ainsi, on peut voir que à la figure 1 la structure porteuse 102 entoure chaque compartiment du fermenteur tandis que le fermenteur est lui-même thermiquement isolé de l'échangeur thermique et de la salle de contrôle 106.

Selon le mode de réalisation actuellement préféré tel que représenté en référence aux figures 7 à 13, l'échangeur thermique est formé par une cuve, de préférence de forme générale sensiblement parallélé-pipèdique, comprenant une paroi médiane 83 séparant l'échangeur en les deux circuits distincts 81, 82 précités; une ou plusieurs cloisons immergées amovibles 84, 86 définissant une pluralité de compartiments 85, 87. Chaque cloison immergée 84, 86 présentant en partie inférieure une échancrure 84a, 86a, visible à la figure 8 de communication entre les compartiments.

Naturellement, chaque circuit 81, 82 comprend respectivement une ouverture d'entrée 88, 90 et de sortie 91, 92 de liquide dans et hors de la cuve formant l'échangeur thermique 80.

Bien sûr, on observera que l'ouverture 91 de sortie du circuit 81 et l'ouverture d'entrée 90 du circuit 82 sont respectivement connectées aux conduites 48 des moyens d'alimentation 4 et 50 des moyens de soutirage 6 de l'effluent liquide relativement au fermenteur 1 tandis que l'ouverture d'entrée 88 et l'ouverture de sortie 92 sont respectivement reliées à l'extérieur par des tuyauteries 94, 95. On observera que toutes les conduites et tuyauteries sont largement dimensionnées afin d'éviter toute possibilité de colmatage par des particules organiques non solubles.

On peut observer à partir de la figure 7 que les cloisons immergées 84, 86 sont maintenues par exemple par des coulisses 96 en forme de U soudées sur la cuve 80 ou sur la cloison médiane 83 et sont ainsi amovibles.

En outre, l'échangeur thermique 80 comprend des moyens d'agitation 110 prévus de manière à réaliser dans chaque compartiment un échange thermique au niveau de la cloison médiane 83.

Ces moyens d'agitation 110 comprennent de préférence une structure rigide 112 comportant deux longerons latéraux 114, 116 montés déplaçables en translation parallèlement à la cloison médiane 83 en reposant sur des galets 118 que l'on voit bien à la figure 11 solidaires de la cuve au moyen de supports 120. Les longerons 114, 116 sont maintenus espacés par des traverses 112 prévues pour enjamber la cloison médiane 83 en position montée. Les traverses 122 portent des pales 124 verticales de préférence profilées en nombre suffisant pour que chaque compartiment défini dans l'échangeur thermique 80 comporte une pale, c'est-à-dire que chaque traverse 122 comprend en fait un couple de pales 124a, 124b situées de part et d'autre de la cloison médiane 83. Les pales 124 sont prévues de manière à laisser un espace libre EL entre leur bord interne situé en regard de la cloison médiane 83 et ladite cloison médiane 83 comme on le voit clairement à la figure 10. D'autre part ces pales 124 sont reliées entre elles à leur partie inférieure par deux éléments rigides 126, 128 qui sont prévus de manière à être disposés de part et d'autre de la cloison médiane étanche 83 et dont la section correspond sensiblement à celle des échancrures 84a, 86a prévues dans les cloisons semi-immergées 84, 86. Lesdits éléments rigides 126, 128 présentent des évidements encoches tels que 130, 132 comme on le voit bien aux figures 9 et 10 séparés par des espacements de longueur identique au pas des cloisons amovibles 84, 86 de manière à ce qu'en fin de course chaque encoche 130, 132 soit située sous chaque cloison immergée amovible 84, 86, comme représenté à la figure 10 afin de permettre le passage de l'effluent liquide entre les compartiments en fin de course ce qui permet d'équilibrer les niveaux d'effluent entre les compartiments. La structure rigide 112 est mûe au moyen d'une bielle 134 qui est par exemple entraînée par un motoréducteur 136 extérieur à l'échangeur thermique 80 mais toujours situé à l'intérieur de l'enceinte isolante 100. Le motoréducteur 136 peut être fixé sur des chevrons 138 qui servent aussi en tant qu'organe de manutention pour l'échangeur complet 80.

Selon une caractéristique particulièrement avantageuse de l'échangeur, la cuve formant l'échangeur 80 est fermée à son sommet par un couvercle amovible 140. Cette cuve comporte par exemple de préférence au niveau de son couvercle 140 un ou plusieurs orifices de raccordement au circuit de biogaz 8; ainsi que des prises pour fixation de sonde de température ou d'échantillon (non représentées). De telles prises sont aussi prévues dans le fermenteur 1. On notera que le circuit de biogaz 8 comprend des conduites 8a et 8b de collecte de biogaz aboutissant respectivement à des robinets 142, 144 situés de préférence en amont de compteurs 146, 148 à gaz dont la sortie est reliée à une conduite unique 150 de biogaz passant au travers de l'enceinte isolante 100 pour aller par exemple à un réservoir de stockage de biogaz (non représenté).

Selon une variante d'exécution, l'échangeur thermique 80 est fermé de manière étanche au fluide et au gaz par un joint thermique de même type que celui utilisé pour le fermenteur 1. Ce mode de réalisation avec le joint thermique est représenté aux figures 12 et 13 et on observera que dans ce cas la cuve formant le fermenteur 80 comporte une rigole 160 sur le pourtour de ses parois externes remplie de liquide et dans ce cas la bielle 134 est modifiée pour présenter une partie en U 161 afin de pouvoir passer de manière convenable dans la rigole 160.

Enfin, de préférence l'installation selon la présente invention comprend aussi des moyens de chauffage d'appoint 170 comprenant de préférence selon le mode de réalisation représenté des plaques chauffantes électriques 172 appliquées contre les parois du fermenteur 1 et de préférence contre les parois du compartiment d'entrée 10 comme représenté; et des moyens de régulation 174 comprenant au moins une sonde thermique 176 et des dispositifs de régulation 178 de préférence disposés dans la chambre de contrôle 106. La chambre de contrôle peut être équipée également de moyens de prise d'échantillon et autres tandis que des purges et mises à l'air sont également prévues dans toute l'installation.

Enfin, on observera en référence à la figure 5 que la tuyauterie 95 de sortie de l'effluent liquide après fermentation et passage dans l'échangeur thermique comprend un élément 95a placé en position réglable plus ou moins verticale constituant ainsi le trop plein de toute l'installation et définissant le niveau réel du liquide (NL).

Le fonctionnement de l'installation ci-dessus décrite est particulièrement simple et est le suivant:

L'effluent liquide brut arrive de préférence en continu par la tuyauterie 94 de fort diamètre, entre dans l'enceinte isolée 100 et pénètre dans l'échangeur thermique 80. Il parcourt le premier circuit 81 et en sort par l'ouverture 91 et la conduite 47 pour aller vers le fermenteur 1 cependant que l'effluent liquide traité sortant du fermenteur 1 parcourt à contre-courant le deuxième circuit 82 de l'échangeur 80 et céde ses calories à travers la cloison médiane 83 qui joue le rôle de paroi d'échange. Les deux circuits 81, 82 sont agités par le mouvement alternatif des pales 124 portées par la structure rigide 112 et se déplaçant chacune entre deux cloisons amovibles successives 84, 86. Les échancrures 84a, 86a des cloisons amovibles 84, 86, respectivement étant normalement obstruées par les éléments rigides 126, 128 à encoches 130, 132 respectivement, chaque compartiment 85, 87 est pratiquement étanche par rapport à ses voisins. Par leur déplacement, les pales 124 forcent le liquide à circuler dans l'espace EL laissé libre au voisinage immédiat de la cloison médiane 83 etaugmentent localement de façon considérable la turbulence des liquides de chaque côté de la cloison médiane 83 qui joue alors le rôle de paroi d'échange très efficace. Cette turbulence locale évite ou limite les phénomènes de décantation, colmatage et/ou encrassement et homogénéise la température du liquide à l'intérieur d'un compartiment donné.

Lorsque la structure rigide 112 supportant les pales 124 et les éléments rigides 126, 128 arrive en fin de course d'un côté de sa trajectoire les évidements 130, 132 de l'élément rigide 112 arrivent au droit des échancrures 84a, 86a des cloisons 84, 86 et laissent une libre circulation des liquides entre deux compartiments successifs 85 ; 87 dont les niveaux tendent à s'égaliser uniquement durant la phase de changement de sens du mouvement des pales 124.

On peut ainsi constater que cet échangeur thermique est particulièrement efficace et qu'il est de conception complètement nouvelle et peut être utilisé en relation avec le fermenteur 1 ou complètement indépendamment de celui-ci dans tous les cas où on désire réaliser un échange thermique avec des fluides liquides ou gazeux entraînant avec eux des particules présentant des risques sérieux de colmatage. Ainsi, les applications d'un tel échangeur thermique sont très variées et non limitées en combinaison au fermenteur 1.

La température souhaitée du fermenteur ou digesteur 1 est de l'ordre de 35°C, par exemple dans le cas d'une flore mésophile. Elle pourrait être notablement supérieure, par exemple de l'ordre de 55°C, dans le cas d'une flore thermophile.

L'effluent liquide à traiter sortant de l'échangeur thermique 80 a acquis une température proche mais inférieure à celle de l'effluent liquide traité sortant du fermenteur 1.

Grâce à l'isolation globale et très efficace de toute l'installation par la présence de l'enceinte isolante 100 et non pas seulement du fermenteur 1, les calories dégagées par tous les équipements électriques (moteur tel que motoréducteur 70 et 136, etc) sont récupérés à peu près intégralement et contribuent à rapprocher encore la température de l'effluent liquide entrant de celle souhaitée dans le fermenteur 1.

Le chauffage final de l'effluent liquide à traiter exige donc une très faible quantité d'énergie et peut sans inconvénient être réalisé à l'aide des moyens de chauffage 170 comprenant des résistances chauffantes 172 appliquées contre les parois du fermenteur 1 et en particulier de son premier compartiment 10 auquel il est très facile d'associer les moyens de régulation 174 permettant d'obtenir une température pratiquement constante dans le fermenteur 1.

L'effluent liquide tel que du lisier, ainsi amené à la température désirée chemine naturellement à travers tous les compartiments successifs du fermenteur 1 en passant par les nombreux canaux des éléments poreux 22 qui sont par exemple constitués par des briques creuses, lesquels canaux se trouvent disposés dans le sens naturel d'écoulement de l'effluent liquide. Les parois des éléments poreux tels que des briques ou analogues, en contact avec le liquide constitue une surface très grande et de structure poreuse particulièrement favorable à la fixation des bactéries ou micro-organismes naturellement présents dans l'effluent liquide tels que du lisier. Dès que ces parois sont raisonnablement saturées en micro-organismes, elles deviennent très actives mais cette activité ne serait pas favorisée du côté phase liquide par la vitesse de déplacement extrêmement lente de celle-ci (temps de séjour dans le fermenteur environ égal à 1 jour habituellement) s'il n'y avait pas une agitation de conception spéciale et qui est fourni par les moyens d'agitation 26.

Cette agitation est obtenue par un mouvement de va-et-vient de la membrane 60 qui induit une pulsation amont-aval dans la partie du circuit comprise entre les deux côtés du système d'agitation, c'est-à-dire pratiquement dans la totalité du fermenteur 1. L'amplitude et la fréquence de cette agitation sont fonction de la structure de l'effluent liquide organique et des éléments poreux mis en place par le fermenteur 1.

On observera qu'avec l'installation selon la présente invention on obtient une accessibilité très aisée. Ainsi, grâce au joint hydraulique et à la conception particulière des supports de bactéries dans le fermenteur d'une part et des moyens d'agitation 110 dans l'échangeur thermique 80 d'autre part il est possible d'accéder à n'importe quel compartiment de l'installation sans manœuvre compliquée et sans devoir ni vidanger l'effluent, ni interrompre le processus de fabrication de biogaz par fermentation anaérobie.

Ainsi, pour le fermenteur 1, le mode opératoire d'accéssibilité est le suivant : on ferme le robinet 142 ou 144 qui met en communication le compartiment visité avec le reste de l'installation et on retire successivement la plaque 104 d'isolation extérieure et le lest éventuel surplombant le compartiment visité et le toit ou couvercle dudit compartiment.

Ainsi, tous les autres compartiments continuent à fonctionner en anaérobie grâce aux cloisons semi-immergées telles que 28, 30, figure 2 qui les séparent et au joint hydraulique tel que 44 indépendant des autres toits amovibles 40. Il est en particulier inutile d'arrêter l'agitation et l'alimentation en effluent liquide de l'installation ce qui est remarquable.

D'autre part, les éléments poreux 22 tels que briques creuses ou analogues du compartiment visité sont accessibles et peuvent être retirés sans difficultés soit pour remplacement soit par nettoyage du colmatage, examen, etc...

La fermeture s'effectue naturellement par remise en place successive du toit 40, du lest éventuel et de la plaque isolante extérieure 104 en vérifiant que le joint hydraulique 44 est suffisamment chargé en liquide.

Avant de ré-ouvrir le robinet de communication 142 ou 144 avec le circuit de biogaz 150 il est prévu de purger l'air emprisonné dans le compartiment au moyen d'un petit robinet non représenté.

De même, pour l'échangeur thermique 80, le mode opératoire initial est le même que celui pour le fermenteur 1. Par contre, il faut arrêter l'agitateur car celui-ci est disposé par dessus la cloison médiane 83 et pour le démonter, il faut retirer au préalable les cloisons amovibles 84, 86 qui sont disposées par dessus les éléments rigides 126, 128 ; désolidariser du mécanisme d'entraînement 134 la structure rigide 112 qui peut alors être dégagée sans difficulté de la cuve de l'échangeur 80.

Les opérations de remontage s'effectuent en ordre inverse.

On comprendra ainsi que l'invention permet d'aboutir aux avantages mentionnés précédemment, à savoir l'obtention d'un continu réel y compris de l'alimentation, d'une récupération de la chaleur de l'effluent liquide sortant du fermenteur 1, l'utilisation de bactéries fixées sur support fixe organisé ce qui accroît la résistance aux agents toxiques ou inhibiteurs, un cheminement horizontal et méthodique de l'effluent liquide tandis que le cheminement du biogaz est différent de celui du substrat liquide, une accessibilité sans vidange du liquide ni interruption du processus permettant le remplacement possible du support pendant et sans perturber le fonctionnement ainsi qu'un contrôle approprié . D'autre part, l'invention permet d'éviter une auto-consommation du biogaz tandis qu'il n'y a plus de colmatage que ce soit dans le fermenteur ou dans l'échangeur thermique.

Naturellement diverses modifications peuvent être apportées par l'homme de l'art et l'invention comprend donc tous les moyens constituant des équivalents techniques des moyens décrits et représentés ainsi que leurs combinaisons. En particulier l'invention est non seulement applicable au lisier mais à tout effluent liquide organique susceptible de subir une fermentation anaérobie. Plus généralement l'installation de l'invention pourrait être appliquée à des fermentations aérobies où se pose le problème de la présence de constituants non solubles dans le liquide à traiter.

Revendications

1. Procédé de production de biogaz à partir d'un effluent liquide organique, tel que par exemple le lisier, du type comprenant une fermentation anaérobie à une température convenable dans un fermenteur étanche contenant des bactéries fixées sur support organisé par alimentation de préférence continue du fermenteur en effluent liquide sans le remplir complètement, soutirage du fermenteur, de préférence continu, de l'effluent fermenté et la récupération du biogaz produit de l'effluent, caractérisé en ce qu'on réalise le fermenteur étanche avec au moins un compartiment de fermentation, chaque compartiment étant pourvu d'une pluralité d'éléments poreux immergés à canaux de passage traversants sensiblement parallèles disposés en piles successives espacées entre elles d'un espace prédéterminé et ayant les canaux en position horizontale et dans le sens d'écoulement naturel de l'effluent liquide dans chaque compartiment, on fait circuler l'effluent liquide horizontalement dans ledit fermenteur, on procède à une agitation, de préférence permanente, de l'effluent liquide avantageusement par pulsation, et on récupère par le sommet du fermenteur le biogaz libéré par la fermentation anaérobie et se dégageant entre les piles d'éléments poreux.

2. Procédé selon la revendication 1, caractérisé en ce qu'on préchauffe l'effluent liquide par échange thermique dans un échangeur thermique disposé à l'extérieur du fermenteur, de préférence l'ensemble fermenteur et échangeur étant disposé à l'intérieur d'une enceinte extérieure thermiquement isolante.

3. Installation de production de biogaz à partir d'un effluent organique liquide, tel que par exemple le lisier, du type comprenant un fermenteur (1) étanche, contenant des bactéries fixées sur un support organisé (2), pourvu de moyens d'alimentation (4) et de moyens de soutirage (6) en effluent liquide, lequel ne remplit pas complètement le fermenteur (1), et des moyens de récupération (8) du biogaz produit de l'effluent, caractérisée en ce que le fermenteur étanche (1) comprend au moins un compartiment (10, 12, 14, 16, 18, 20) de fermentation pourvu d'une pluralité d'éléments poreux (22) immergés à canaux de passage traversants sensiblement parallèles disposés en piles successives, telles que (23, 24) espacées entre elles d'un espace prédéterminé (e) et ayant leurs canaux en position horizontale et dans le sens d'écoulement naturel de l'effluent liquide circulant horizontalement dans le fermenteur (1) ; les moyens de récupération de biogaz (8) comportant un ou plusieurs conduits (8a, 8b) débouchant au sommet de chaque compartiment pour collecter le biogaz libéré dégagé entre les piles d'éléments poreux (22) ; et de préférence des moyens (26) avantageusement d'agitation permanente, de l'effluent liquide de préférence en réalisant des pulsations.

4. Installation selon la revendication 3, caractérisée en ce qu'elle comprend un échangeur thermique (80) disposé à l'extérieur du fermenteur (1) dont le circuit de fluide réfrigérant (81) est parcouru par l'effluent liquide à introduire dans le fermenteur (1) et le circuit de fluide chauffant (82) est parcouru par l'effluent liquide sortant du fermenteur (1), de préférence l'ensemble fermenteur (1)-échangeur thermique (80) étant disposé à l'intérieur d'une enceinte (100) fermée thermiquement isolante.

5. Installation selon la revendication 3 ou 4, caractérisée en ce que le fermenteur comprend une pluralité de compartiments successifs (10, 12, 14, 16, 18, 20) indépendants dont la paroi commune (telle que 28, 30) entre deux compatiments successifs est au moins en partie ouverte vers le bas définissant ainsi une ouverture (telle que 36, 38) respectivement de passage de l'effluent liquide, chaque compartiment comportant un toit amovible (40) propre de fermeture étanche reposant sur les parois de son compartiment associé (10).

6. Installation selon la revendication 5, caractérisée en ce que chaque compartiment (10, 12, 14, 16, 18, 20) comprend un joint hydraulique, tel que (44), assurant l'étanchéité entre le toit (40) et les parois du compartiment associé permettant une ouverture indépendante des compartiments.

7. Installation selon l'une quelconque des revendications 3 à 6, caractérisée en ce que le fermenteur est une cuve sensiblement parallélépipèdique comportant une cloison médiane (27) étanche de séparation du fermenteur (1) en deux zones et une ou plusieurs cloisons telles que (28, 30) semi-immergées ouvertes vers le bas définissant une pluralité de compartiments successifs précités ayant chacun un toit propre (40) le compartiment d'entrée (10) de l'effluent et le compartiment de sortie (20) de l'effluent étant situés de part et d'autre de la cloison médiane (27) qui comporte une ouverture (46) de communication entre les compartiments (14, 16)) les plus éloignés desdits compartiments d'entrée (10) et de sortie (20) d'effluent.

8. Installation selon la revendication 7, caractérisée en ce que les moyens d'agitation précités sont formés par une membrane (60) souple et déformable fixée sur une ouverture (62) prévue dans la paroi médiane (27) entre deux compartiments, de préférence les comparti- ments d'entrée (10) et de sortie (20) de l'effluent liquide, rendue rigide en son centre par un disque (64) mû suivant son axe par un mécanisme approprié (66) pour créer des pulsations amont-aval dans le fermenteur.

9. Installation selon l'une quelconque des revendications 4 à 8, caractérisée en ce que l'échangeur thermique (80) est formé par une cuve comprenant une paroi médiane étanche (83) séparant l'échangeur en deux circuits distincts (81, 82) , une ou plusieurs cloisons immergées amovibles (84, 86) définissant une pluralité de compartiments (85, 87) distincts présentant en partie inférieure une échancrure (84a, 86a) de communication entre les compartiments ; chaque circuit comprenant une ouverture d'entrée (88, 90) respectivement et de sortie (91, 92) respectivement de fluide dans et hors de la cuve et des moyens d'agitation (110) sont prévus pour favoriser dans chaque compartiment un échange thermique au niveau de la cloison médiane (83).

10. Installation selon la revendication 9, caractérisée en ce que la cuve de l'échangeur (80) est fermée à son sommet par un couvercle amovible (140) laissant un espace libre au-dessus des compartiments et de la cloison médiane (83), les moyens d'agitation (110) comprennent de préférence une structure rigide comportant deux longerons (114, 116) latéraux montés déplaçables en translation parallèlement à la cloison médiane (83), lesdits deux longerons (114, 116) étant montés espacés par des traverses (122) enjambant la cloison médiane (83) en position montée, lesdites traverses (122) portant des pales (124) verticales profilées de sorte que chaque compartiment comporte une pale, lesdites pales (124) laissant un espace libre EL entre leur bord intérieur situé en regard de la cloison médiane (83) et ladite cloison médiane (83), lesdites pales (124) sont reliées entre elles à leur partie inférieure par deux éléments rigides (126, 128) dont la section correspond sensiblement à celle des écharcrures (84a, 86a), lesdits éléments rigides (126, 128) présentant des évidements encoches (130, 132) séparés par des espacements de longueur identique au pas des cloisons amovibles (84, 86) de manière à ce qu'en fin de course chaque encoche (13o, 132) soit située sous chaque cloison immergée (84, 86) respectivement.

11. Echangeur thermique, caractérisé en ce qu'il constitue un moyen nécessaire pour la mise en œuvre du procédé selon la revendication 1 ou 2 ou de l'installation selon l'une quelconque des revendications 4 à 10 et en particulier en ce qu'il présente la structure telle que défini à la revendication 9 ou 10.

FIG. 1

**Fig. 2**

IV  170  172  23  e  24  1  12  14

2  -10-

62  47  22

III  60  26  4  48

IV  20  70  18  16  27  III

**Fig. 3**

10  40  45  1

41  44  41  28  30

42

60  -22-  e -22-  36  38

-22-  -22-

-22-  -22-

48  64  23  24

**Fig. 4**

10  8  8b

40  40  8a

62  60  26  -22-  66  68

64

-22-  1

1

0102279

0102279

**Fig:5**

**Fig:6**

**Fig:7**

**Fig:8**

**Fig:9**

0102279

## Fig. 10

124b  84  130  126  83  EL  EL  86  128  124a  132

## Fig. 11

140  115  115  136  120  90  91

## Fig. 12

134  160  XIII  136  138  161

## Fig. 13

140  115  115  115  136

Office européen
des brevets

**RAPPORT DE RECHERCHE EUROPEENNE**

EP 83 40 1581

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | DE-A-2 951 324 (SAUFF H. WERNER) | | C 12 M 1/00 |
| A | EP-A-0 033 017 (SOCIETE NATIONALE ELF AQUITAINE) | | |
| A | FR-A-1 082 647 (DA COSTA) | | |

| DOMAINES TECHNIQUES RECHERCHES (Int. Cl. ³) |
|---|
| C 12 M |

Le présent rapport de recherche a été établi pour toutes les revendications

| Lieu de la recherche | Date d'achèvement de la recherche | Examinateur |
|---|---|---|
| LA HAYE | 08-11-1983 | COUCKE A.O.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82